**Europäisches Patentamt**

**European Patent Office**

**Office européen des brevets**

(11) Veröffentlichungsnummer: **0 000 134 B1**

(12) **EUROPÄISCHE PATENTSCHRIFT**

(45) Veröffentlichungstag der Patentschrift:
**28.10.81**

(21) Anmeldenummer: **78100134.2**

(22) Anmeldetag: **09.06.78**

(51) Int. Cl.³: **C 07 G 7/00, G 01 N 33/48**

(54) **Glykoprotein, Verfahren zu seiner Herstellung, seine Verwendung zur Gewinnung von Antiserum sowie das Antiserum.**

(30) Priorität: **15.06.77 DE 2726886**

(43) Veröffentlichungstag der Anmeldung:
**10.01.79 Patentblatt 79/1**

(45) Bekanntmachung des Hinweises auf die Patenterteilung:
**28.10.81 Patentblatt 81/43**

(84) Benannte Vertragsstaaten:
**BE CH DE FR GB LU NL SE**

(56) Entgegenhaltungen:
**DE-A-2 256 168**
**FR-A-2 183 151**
**Chemical Abstracts, vol. 69, 103100m (1968)**

(73) Patentinhaber: **BEHRINGWERKE AKTIENGESELLSCHAFT, Postfach 1140, D-3550 Marburg/Lahn (DE)**

(72) Erfinder: **Bohn, Hans, Dr., Oberer Eichweg 26, D-3550 Marburg/Lahn (DE)**
Erfinder: **Winckler, Wilhelm, Am Hang 23, D-3551 Wenkbach (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

Glykoprotein, Verfahren zu seiner Herstellung,
seine Verwendung zur Gewinnung von Antiserum, sowie das Antiserum

Die Erfindung betrifft ein neues Glykoprotein, das im Blutserum, im Urin und im Extrakt menschlicher Plazenten nachgewiesen und daraus isoliert werden kann, sowie ein Verfahren zu dessen Herstellung.

Die durch die wässrige Extraktion menschlicher Plazenten erhältliche Proteinlösung enthält bekanntlich eine Vielzahl von Komponenten, die teilweise den Serumproteinen zuzuordnen und zum anderen Teil Gewebeproteine sind.

Die Erfindung hat sich die Aufgabe gestellt, ein bisher noch nicht bekanntes Serum-Glykoprotein aus dem Extrakt menschlicher Plazenten zu isolieren, damit spezifisch gegen das neue Glykoprotein gerichtete Antiseren herzustellen, womit das Glykoprotein im Serum und im Urin qualitativ nachgewiesen oder quantitativ bestimmt werden kann.

Gegenstand der Erfindung ist ein neues Glykoprotein, welches aus Blutserum, aus Urin und dem Extrakt menschlicher Plazenten erhältlich ist. Es ist gekennzeichnet durch einen Proteinanteil, im wesentlichen bestehend aus $\alpha$-Aminosäuren von $75\pm 6\%$, einen Kohlenhydratanteil von $24,6 \pm 5,2\%$, davon Hexosen $8,9 \pm 2\%$, N-acetyliertes Hexosamin $7,1 \pm 1,5\%$, Fucose $0,2\pm 0,2\%$, N-acetylierte Neuraminsäure $8,4 \pm 1,5\%$, einen Sedimentationskoeffizienten $s_{20w}$ von $2,5 \pm 0,3$ S, ein Molekulargewicht von $35\,000 \pm 5\,000$ aufgrund der Bestimmung in der Ultrazentrifuge bzw. ein Molekulargewicht von $65\,000 \pm 10\,000$ aufgrund der Bestimmung im Natriumdodezylsulfat-haltigen Polyacrylamidgel, einen isoelektrischen Punkt von pH $3,4 \pm 0,4$, einen Extinktionskoeffizienten $E_{1cm}^{1\%}$ (280 nm) von $1,9\% \pm 0,3$, eine elektrophoretische Beweglichkeit im Bereich zwischen den $\alpha_1$- und den $\alpha_2$-Globulinen und eine spezifische immunologische Reaktion mit einem spezifisch gegen das Glykoprotein gerichteten Antikörper.

Zur Erläuterung der kennzeichnenden Merkmale des Glykoproteins sei folgendes ausgeführt:

Die Bestimmung der Sedimentationskoeffizienten wurde in einer analytischen Ultrazentrifuge der Firma Beckman (Spinco-Apparatur, Modell E) bei 6000 UpM in Doppelsektorzellen mit Hilfe der UV-Scanner Technik bei 280 nm durchgeführt. Als Lösungsmittel diente ein 0,05 M Phosphatpuffer (pH 6,8) der 0,2 Mol/l NaCl enthielt. Die Proteinkonzentration betrug 2%. Die Sedimentationskoeffizienten sind auf die Basis von Wasser bei 20 °C umgerechnet worden.

Zur Ermittlung der Molekulargewichte wurde die Sedimentationsgleichgewichtsmethode und die Polyacrylamidgel-Elektrophorese herangezogen. Die Bestimmung in der Ultrazentrifuge wurde bei 9000 UpM vorgenommen. Die Auswertung erfolgte unter Zugrundelegung eines partiellen spezifischen Volumens (partial specific volume) von 0,74 mg/g. In der Ultrazentrifuge ergab sich ein Molekulargewicht von $35\,000 \pm 5000$.

Für die Polyacrylamidgel-Elektrophorese wurden zwei Verfahren angewandt. Die Auftrennung im normalen Polyacrylamid (PAA)-Gel erfolgte nach der Methode von Zwisler und Biel, Z.klin. Chem. 4, Seite 58, (1966). Zur Untersuchung im Natriumdodecylsulfat-haltigen Gel wurde ein Gel mit 7,5% PAA, das 0,1% Natriumdodecylsulfat (SDS) enthielt, verwendet. Zur Reduktion sind die Proteine in 1% SDS mit 1% Merkaptoäthanol inkubiert worden. Das Anfärben der Proteine geschah mit Amidoschwarz. Aus der Wanderung im SDShaltigen PAA-Gel wurde für das Glykoprotein ein Molekulargewicht von $65\,000 \pm 10\,000$ abgeleitet.

Die Bestimmung des isoelektrischen Punktes wurde mit einer Säule (440 ml) der Firma LKB Stockholm, durchgeführt. Das sogenannte Ampholin-Gemisch hatte bei der Untersuchung des Glykoproteins einen pH-Bereich von 2,5 bis 4,0.

Die Untersuchung der elektrophoretischen Beweglichkeit erfolgte in der Mikromodifikation von Beckmann Instruments auf Zelluloseazetatfolien mit Natriumdiäthylbarbiturat-Puffer pH 8,6.

Die Bestimmung der Kohlehydrate erfolgte nach der von H. E. Schultze, R. Schmidtberger, H. Haupt, Biochem. Z. 329, Seite 490, (1958), beschriebenen Methode.

Die Aminosäurenanalyse wurde nach S. Moore, D. H. Spackmann, W. H. Stein, Anal. Chem. 30, Seite 1185, (1958), unter Verwendung des Flüssigkeitschromatographen Multichrom B der Firma Beckmann durchgeführt. 1/2 Cystin wurde nach Oxydation der Proteine mit Perameisensäure (S. Moore et al., Anal. Chem. 30, Seite 1185, (1958) ) und nachfolgender Chromatographie (S. Moore, J. Biol. Chem. 238, Seite 235, (1963) ) als Cysteinsäure bestimmt. Der Tryptophangehalt ist mit der direkten photometrischen Bestimmung nach H. Edelhoch, Biochemistry 6, Seite 1948, (1967) ermittelt worden.

Die immunologische Charakterisierung der Substanz erfolgt am einfachsten durch ein bekanntes Diffusionsverfahren, bei welchen Antigen, d.h. das Glykoprotein und ein gegen das Glykoprotein gerichteter Antikörper bzw. das hinsichtlich der Antikörper nicht angereicherte Antiserum gegeneinander in einem Trägermedium, wie Agar, diffundieren. Treffen die beiden Reaktionskomponenten in einem günstigen Verhältnis aufeinander, bildet sich ein sichtbares Präzipitat aus. Nach dieser Kenntnis ist es für den Fachmann einleuchtend, dass alle immunologischen Techniken zum Nachweis und zur Bestimmung sowohl des neuen Glykoproteins, als auch der gegen das Glykoprotein gerichtete Antikörper möglich sind.

Eine einfache und in der Regel ausreichend genaue Methode zur quantitativen Bestimmung des Glykoproteins in Körperflüssigkeiten oder in Gewebeextrakten stellt die sogenannte Laurell-Technik dar. Sie ist beschrieben in Analyt. Biochem. (New York), 15, Seite 45 (1966).

Gegenstand der Erfindung ist ferner ein Verfahren zur Herstellung des oben charakterisierten Glykoproteins, dadurch gekennzeichnet, dass Körperflüssigkeiten oder Extrakte von Organen, welche das Glykoprotein enthalten, unter Zugrundelegung folgender erfindungsgemäss gefundener Kriterien fraktioniert werden.

Das Glykoprotein ist mit Neutralsalzen fällbar. Mit dem üblicherweise für derartige Fällungen verwendeten Ammoniumsulfat wird es bei einer Sättigungskonzentration des Salzes von 30 bis 60% in einem pH-Bereich in der Nähe des Neutralpunktes gefällt.

Entsprechend seinem Molekulargewicht kann das Glykoprotein durch Massnahmen, die zur Abtrennung von Substanzen mit Molekulargewichten zwischen 25 000 und 75 000 geeignet sind, gewonnen werden. Vorteilhaft werden hierfür die Methoden der Gel-Filtration oder Ultrafiltration eingesetzt.

Das Glykoprotein wird bei neutralem oder schwach alkalischem pH-Wert an schwach basische Ionenaustauscher adsorbiert. Vorteilhaft wird dabei eine vergleichsweise wenig konzentrierte Pufferlösung verwendet, denn durch Erhöhung der Salzkonzentration oder auch durch Erniedrigung des pH-Wertes kann die Adsorption verhindert werden. Andererseits bietet sich bei Kenntnis dieses Verhaltens die Möglichkeit an, das Glykoprotein zu adsorbieren und unter Verwendung von höherkonzentrierten Salzlösungen bzw. von Pufferlösungen mit erniedrigtem pH-Wert zu eluieren.

Es hat sich gezeigt, dass das neue Glykoprotein mit den wasserlöslichen organischen Basen der Acridin- und Chinolinreihe, die für Protein-Fällungsverfahren üblicherweise Verwendung finden, nicht präzipitiert wird. Es bleibt in den bei diesem Verfahren üblichen Konzentrationen im wässrigen Überstand. Danach kann eine Acridinbase, wie 2-Äthoxy-6,9-Diaminoacridinlactat oder eine Chinolinbase, wie Bis-(2-methyl-4-aminochinolyl-6)-carbamid-hydrochlorid, zur Fällung von begleitenden Proteinen verwendet werden, wobei das erfindungsgemässe Glykoprotein im Überstand verbleibt.

Ähnliche Überlegungen können gelten bei Verwendung von Hydroxylapatit als Adsorbens für Proteine. Das neue Glykoprotein zeigt keine Affinität zum Hydroxylapatit, wohingegen eine Reihe von Begleitproteinen von Hydroxylapatit festgehalten wird. Das Glykoprotein gehört somit zu den Hydroxylapatit-passierenden Globulinen. Der Erfinder schlägt vor, es als Hydroxylapatit passierendes Globulin (HPG-2) zu bezeichnen.

Aufgrund der Kenntnis der elektrophoretischen Beweglichkeit kann für die Anreicherung bzw. Isolierung des Glykoproteins die präparative Zonenelektrophorese eingesetzt werden.

Die Affinität des Glykoproteins aufgrund seines immunologischen Verhaltens kann dafür eingesetzt werden, das Glykoprotein mit Hilfe von sog. Immun-Adsorptionsverfahren anzureichern. Hierfür kann in an sich bekannter Weise ein Immunadsorbens d.h. ein trägergebundener Antikörper, gegen das neue Glykoprotein hergestellt werden, welches das Glykoprotein spezifisch zu binden vermag. Das Glykoprotein kann danach durch Änderung der Milieubedingungen wieder eluiert werden, wie dies mehrfach beschrieben ist.

Durch eine ausgewählte Kombination der genannten Methoden, die einerseits zur Anreicherung des Glykoproteins, andererseits zu einer Abtrennung von übrigen Begleitproteinen führen, kann die Isolierung der erfindungsgemässen Substanzen erfolgen. Demzufolge ist der Gegenstand der vorliegenden Erfindung in den einzelnen Anreicherungsschritten für das neue Glykoprotein und in den durch Kombination der Massnahmen zur Anreicherung sich ergebenden Verfahren zu dessen Reinigung zu sehen.

Die Leitlinie für das Verfahren zur Herstellung des Glykoproteins besteht darin, dass jeweils derjenige Anteil gewonnen wird, welcher eine positive immunologische Reaktion mit einem gegen das neue Glykoprotein gerichteten Antiserum ergibt.

Nach Durchführung der genannten Verfahrensschritte zeigt es sich zuweilen, dass das Glykoprotein noch von anderen immunologisch nachweisbaren Begleitproteinen verunreinigt ist. In diesem Falle werden die Verunreinigungen durch deren spezifische Adsorption entfernt. Man bedient sich dabei gängiger Techniken der Immunadsorption, bei welchen nach beschriebenen Verfahren an einen Träger gebundene Antikörper gegen das zu entfernende Protein als Adsorbenzien eingesetzt werden. Häufig ist das weitgehend reine neue Glykoprotein noch mit Spuren des schwangerschafts-spezifischen $\beta_1$-Glykoproteins und/oder des $\alpha_1$-B-Glykoproteins, auch als leicht fällbares $\alpha_1$-Glykoprotein bezeichnet, vorgesellschaftet. Zu deren Abtrennung können gegen die Proteine gerichteten Immunoglobuline, welche kovalent an vernetzte Agarpräparation, z.B. Sepharose, gebunden sind, verwendet werden.

Die auf eine Säule, welche mit dem spezifischen Immunadsorbens gefüllt wurde, aufgetragene Proteinlösung läuft insoweit unbehelligt durch die Säule, als nur diejenigen Komponenten gebunden werden, gegen die der Träger einen immunologisch aktiven Partner enthält. Das neue Glykoprotein kann auf diese Weise von den Verunreinigungen befreit werden.

Zur Herstellung des neuen Glykoproteins werden mehrere der angeführten Massnahmen miteinander kombiniert und dabei jeweils diejenige Fraktion weiterverarbeitet, in der immunologisch das neue Glykoprotein nachgewiesen werden kann, während die übrigen Fraktionen verworfen werden.

Als Ausgangsmaterial für die Herstellung des neuen Glykoproteins kann jede Körperflüssigkeit oder jeder Organextrakt verwendet werden, in welchem es gelingt, das Glykoprotein immunologisch nachzuweisen. Bevorzugt werden Extrakte menschlicher Plazenten verwendet, die durch Zerkleinerung und Extraktion mit Wasser oder einer verdünnten, zweckmässig einer weniger als 10%igen Salzlösung, vorteilhaft mit einer 0,5%igen Neutralsalzlösung, wie Natriumchlorid, gewonnen werden können. Zweckmässig verwen-

det man auf 1 kg Plazenten etwa 1–5 Liter der Extraktionslösung. Die nicht gelösten Anteile werden durch Zentrifugation oder Filtration von dem Extrakt abgetrennt.

Das Verfahren zur Anreicherung ist gekennzeichnet durch die Anwendung mindestens einer der folgenden Massnahmen auf Körperflüssigkeiten, welche das neue Glykoprotein enthalten und die anschliessende Gewinnung der bezüglich des Glykoproteins angereicherten Fraktion:

a) Zugabe von wasserlöslichen Derivaten einer Acridin- oder Chinolinbase, vorzugsweise des 2-Äthoxy-6,9-Diaminoacridin-lactat, im pH-Bereich von 5–10, vorzugsweise bei etwa pH 8, bis zu einer Endkonzentration von etwa 0,8% (Gewicht zu Volumen), wobei das Glykoprotein im wesentlichen im Überstand verbleibt.

b) Zugabe von Neutralsalzen bis zur Ausfällung des Glykoproteins, vorzugsweise von Ammoniumsulfat, bei etwa neutralem pH-Wert von 5–8 bis zu 30–60% der Sättigungskonzentration des Ammoniumsulfats.

c) Adsorption des Glykoproteins an einem schwach-basischen Ionenaustauscher, wie Diäthylaminoäthyl-cellulose, bei einer Leitfähigkeit der Lösung von 0–2 mS und neutralem oder schwachalkalischem pH-Wert (6–9). Beispielsweise unter Verwendung eines etwa 0,01 M Puffers vom pH-Wert von etwa 8. Ein bevorzugt zu verwendender Puffer ist Tris-hydroxymethylaminomethan-HCl. Die Elution des Glykoproteins kann durch Senkung des pH-Wertes unter pH 7,0 oder durch Erhöhung der Leitfähigkeit über 5 mS erreicht werden.

d) Trennung aufgrund der Molekülgrösse (Molekularsiebfraktionierung). Besonders geeignet ist die Gelfiltration in einer Säule gefüllt mit einem Polymeren entsprechender Porengrösse, beispielsweise mit Epichlorhydrin-vernetztem Dextran, als Sephadex® hergestellt von der Firma Pharmacia, Uppsala, mit dem Ziel der Anreicherung von Proteinen mit einem Molekulargewicht von etwa 50 000. Aber auch Produkte wie Ultrogel® von LKB, Bromma oder Bio-Gel® von Bio-Rad Laboratories, Richmond, Calif. können eingesetzt werden.

e) Durchführung einer Adsorption mit Hydroxylapatit. Da das Glykoprotein in verdünnter Phosphatpufferlösung von Hydroxylapatit nicht gebunden wird, ist Hydroxylapatit ein geeignetes Agens, um Begleitproteine des Glykoproteins aus der Lösung zu entfernen. Die Proteinlösung wird hierfür zweckmässig auf einen pH-Wert um den Neutralpunkt eingestellt und die Leitfähigkeit der Lösung auf etwa 1 mS gehalten.

f) Präparative Zonenelektrophorese.

Geeignet für die Durchführung einer Elektrophorese ist eine das Glykoprotein enthaltende Lösung, vorzugsweise eine alkalische Pufferlösung, beispielsweise in einem Natriumdiäthylbarbituratpuffer von pH 8,6, und einer Ionenstärke=0,1. Die Lösung wird in eine Apparatur zur präparativen Elektrophorese eingetragen, wie sie beispielsweise von N. Heimburger und R. Schmidtberger in Behringwerke-Mitteilungen, Heft 43, Seite 83 ff.,

insbesondere auf Seite 119–120, beschrieben wird. Bei dem Gerät handelt es sich um die horizontale Anordnung einer Trägerelektrophorese in einem offenen Trog, in welchem das Trägermaterial zur Abführung der bei der Elektrophorese auftretenden Joul'schen Wärme auf unter 10 °C gekühlt wird. Als Trägermaterial dienen gegenüber Proteinen indifferente Substanzen, vorteilhaft Polyvinylchlorid, oder dessen Copolymerisate in Form eines Granulats.

Es ist empfehlenswert, die Elektrophorese im alkalischen pH-Bereich, vorteilhaft bei etwa pH 8,6, bei einer Ionenstärke von 0,08–0,12 und bei einer Feldstärke von 4–6 Volt/cm vorzunehmen. Bei Verwendung von 0,1 M Natriumdiäthylbarbituratpuffer vom pH-Wert 8,6 wandert das Glykoprotein im elektrischen Feld in den zwischen $\alpha_1$- und $\alpha_2$-Globulinen liegenden Bereich der Plasmaproteine.

Für die Gewinnung des neuen Glykoproteins wird die betreffende Zone herausgeschnitten und vom inerten Trägermaterial mit Hilfe von Wasser oder wässriger Salzlösungen, beispielsweise einer 0,5 bis 1%igen Kochsalzlösung, eluiert.

Das erfindungsgemäss hergestellte Glykoprotein hat antigene Eigenschaften. Eine damit durchgeführte Immunisierung von Tieren nach bekannten Methoden führte zur Bildung von spezifischen Antikörpern im Blut der immunisierten Tiere. Deren Seren können nach üblichen Verfahren gewonnen und die darin enthaltenen Antikörper angereichert werden. Die Antiseren können in bekannten immunologischen Verfahren zum Nachweis und zur Bestimmung des neuen Proteins in Körperflüssigkeiten, insbesondere in dem Blutserum, Verwendung finden.

Die Erfindung wird in dem nachstehenden Beispiel näher erläutert.

Beispiel

150 kg tiefgefrorene Plazenten werden zerkleinert und mit 150 l einer 0,5%igen wässrigen Natriumchloridlösung extrahiert. Der Extrakt wird mit 2n-Natriumhydroxyd auf pH 8 eingestellt und mit 50 l einer 3%igen wässrigen Lösung von Diaminoäthoxyacridin-lactat versetzt. Nach einer Wartezeit von 1 Stunde wird der Überstand der das erfindungsgemässe Glykoprotein (HPG-2) enthält, abgehebert, mit 5% festem Natriumchlorid (11 kg) zur Abscheidung des noch in Lösung verbliebenen Diaminoäthoxyacridin-lactats versetzt, filtriert und mit 30% – bezogen auf das Gewicht der Flüssigkeit – festem Ammoniumsulfat versetzt und gut durchgerührt. Nach 1 Stunde wird der Niederschlag abfiltriert.

500 g des auf dem Filter gesammelten Niederschlages werden in 500 ml destilliertem Wasser gelöst und gegen eine 0,01 molare Tris-(hydroxymethyl)-aminomethan-Salzsäure-Pufferlösung vom pH-Wert 7,0, die 0,05% Natriumazid enthält, dialysiert. Die dialysierte Lösung wird zentrifugiert und der Überstand wird mit der gleichen Pufferlösung auf 2000 ml aufgefüllt, mit 0,1 n Natriumhydroxydlösung auf pH 8,0 eingestellt und mit 500 g feuchter Diäthylaminoäthylcellulose (Firma Serva, Heidelberg) 1 Stunde verrührt.

Dann wird die Diäthylaminoäthylcellulose durch Filtrieren von der Lösung abgetrennt, zweimal mit je 1 Liter 0,01 molarem Tris-(hydroxymethyl)-aminomethan-Salzsäure-Puffer vom pH-Wert 8,0 gewaschen und danach dreimal mit je 500 ml 0,02 molarem Tris-(hydroxymethyl)-aminomethan-Salzsäure-Puffer, pH 6,5, der 0,85% Natriumchlorid und 0,05% Natriumazid enthält, eluiert.

Den vereinigten Eluaten werden 30% Ammonsulfat, bezogen auf das Flüssigkeitsgewicht, zugesetzt und das ganze wird verrührt. Der Niederschlag, der das Protein (HPG-2) enthält, wird in 300 ml destilliertem Wasser gelöst. Die Proteinlösung wird gegen Tris-hydroxymethyl-Aminomethan-Salzsäure-Puffer von pH 8,0, der 1,0 Mol Natriumchlorid/l enthält, dialysiert und auf eine mit Sephadex G-150 gefüllte Säule (100×20 cm) aufgetragen und mit dem genannten Puffer eluiert. Während der Elution findet eine Fraktionierung der Proteine nach deren Molekülgrösse statt.

Anschliessend werden die Eluate mit spezifischem Antiserum getestet, die das Glykoprotein (HPG-2) enthaltenden Fraktionen werden gesammelt und daraus die Proteine nochmals, wie oben beschrieben, mit 30% festem Ammonsulfat ausgefällt.

Zur Weiterreinigung wird die Fällung in 50 ml Wasser gelöst, gegen einen 0,005 m Phosphatpuffer, pH 6,8, dialysiert und auf eine mit Hydroxylapatit gefüllte Säule, 3×23 cm, gegeben. Die Entwicklung der Säule erfolgt mit dem 0,005 m Phosphatpuffer, pH 6,8.

Das Glykoprotein (HPG-2) erscheint im Durchlauf; dieser wird auf einem Ultrafilter eingeengt. Das Konzentrat wird anschliessend gegen einen 0,01 M Tris-HCl-Puffer, pH 7,0, dialysiert und an Deae-Sephadex (Säule 3× 23 cm) adsorbiert. Zur Elution und Auftrennung der adsorbierten Proteine dient ein NaCl-Gradient von 0–2%. Die das Glykoprotein (HPG-2) enthaltenden Eluatfraktionen werden gesammelt eingeengt.

Zur Weiterreinigung wird das eingeengte Eluat in einer 0,075 m Ammoniumbikarbonatlösung aufgenommen und einer präparativen Zonenelektrophorese unterworfen. Die das HPG-2 enthaltende Zone wird nach der Auftrennung herausgeschnitten und mit physiologischer Kochsalzlösung eluiert; die Eluate engt man anschliessend auf dem Ultrafilter ein.

Das als Verunreinigung noch vorhandene $\alpha_1$B-Glykoprotein wird mit Hilfe eines entsprechenden Immunadsorbens entfernt. Zur Herstellung des Immunadsorbens werden Antikörper gegen das $\alpha_1$B-Glykoprotein kovalent an Sepharose gebunden und das so erhaltene Adsorbens mit dem Eluat im Batch-Verfahren oder in einer Säule in Berührung gebracht. Dabei wird das $\alpha_1$B-Glykoprotein an die trägergebundenen Antikörper adsorbiert, während das Glykoprotein HPG-2 in Lösung bleibt. Die Lösung, die dann nur noch HPG-2 enthält, wird gegen Wasser dialysiert und lyophilisiert. Man erhält etwa 10 bis 30 mg des neuen Glykoproteins HPG-2.

Es zeigt folgende Aminosäure-Zusammensetzung (Häufigkeit mit Variationskoeffizienten (VK in %):

| | Häufigkeit in Mol % | VK % |
|---|---|---|
| Lysin | 4,41 | 6,92 |
| Histidin | 1,22 | 22,79 |
| Arginin | 1,34 | 9,18 |
| Asparaginsäure | 8,23 | 2,56 |
| Threonin | 6,74 | 2,42 |
| Serin | 5,26 | 6,85 |
| Glutaminsäure | 12,59 | 0,49 |
| Prolin | 11,27 | 9,20 |
| Glycin | 5,63 | 9,84 |
| Alanin | 15,05 | 6,35 |
| Cystin/2 | 3,65 | 22,76 |
| Valin | 9,89 | 7,94 |
| Methionin | 0,0 | 0,0 |
| Isoleucin | 0,88 | 7,39 |
| Leucin | 9,09 | 3,74 |
| Tyrosin | 1,02 | 30,77 |
| Phenylalanin | 3,60 | 5,14 |
| Tryptophan | 0,14 | 96,98 |

Das Protein kann in ähnlicher Weise aus Serum, auch aus Männerserum, gewonnen werden. Dazu werden als Ausgangsmaterial 100 Liter Serum eingesetzt, das mit 100 l destilliertem Wasser verdünnt wird. Das Protein wird analog Beispiel 1 mit Rivanol gefällt und weitergereinigt.

**Patentansprüche**

1. Glykoprotein gekennzeichnet durch
a) einen Proteinanteil von 75 ±6%;
b) einen Kohlenhydratanteil von 24,6 ± 5,2%, davon Hexosen 8,9 ± 2%, N-acetyliertes Hexosamin 7,1 ± 1,5%, Fucose 0,2 ± 0,2%, N-acetylierte Neuraminsäure 8,4 ± 1,5%;
c) einen Sedimentationskoeffizienten $S_{20w}$ von 2,5 ± 0,3 S;
d) ein in der Ultrazentrifuge bestimmtes Molekulargewicht von 35 000 ± 5000;
e) einen isoelektrischen Punkt von pH 3,4 ± 0,4;
f) einen Extinktionskoeffizienten $E_{1cm}^{1\%}$ (280 mm) von 1,9 ± 0,3;
g) eine elektrophoretische Beweglichkeit im Bereich zwischen $\alpha_1$- und den $\alpha_2$-Globulinen;
h) eine spezifische immunologische Reaktion mit einem spezifisch gegen das Glykoprotein gerichteten Antikörper.

2. Verfahren zur Anreicherung des Glykoproteins nach Anspruch 1, dadurch gekennzeichnet, dass eine Proteinlösung, die das Glykoprotein enthält, mindestens einer der folgenden Massnahmen unterworfen wird und die bezüglich des Glykoproteins angereicherte Fraktion gewonnen wird:
a) Zugabe von Neutralsalzen bis zur Ausfällung des Glykoproteins;

b) Molekularsiebfraktionierung und Gewinnung der Fraktion mit einem Molekulargewicht von 25 000 bis 75 000;
c) Adsorption des Glykoproteins an einen schwach basischen Ionenaustauscher und Elution davon;
d) Zugabe von wasserlöslichen Derivaten einer Acridin oder Chinolinbase im Bereich von pH 5–10 bis zu einer Endkonzentration von etwa 0,8%;
e) Behandlung der Glykoproteinlösung mit Hydroxylapatit;
f) Präparative Zonelektrophorese und Gewinnung der Zone zwischen $\alpha_1$- und $\alpha_2$-Globulinen;
g) Behandlung der Proteinlösung mit einem Immunadsorbens.

3. Verfahren nach Anspruch 2, dadurch gekennzeichnet, dass als Proteinlösung ein Extrakt aus menschlichen Plazenten verwendet wird.

4. Verwendung des Proteins nach Anspruch 1 zur Gewinnung von Antiseren.

5. Antiserum erhalten durch Immunisierung von Wirbeltieren mit einem Glykoprotein nach Anspruch 1.

**Revendications**

1. Glycoprotéine, caractérisée par:
a) une teneur en protéines de 75 ± 6%;
b) une teneur en hydrates de carbone de 24,6 ± 5,2%, dont hexoses 8,9 ± 2%, n-acétyl hexosamine 7,1 ± 1,5%, fucose 0,2 ± 0,2%, acide N-acétyl neuraminique 8,4 ± 1,5%;
c) un coefficient de sédimentation $S_{20w}$ de 2,5 ± 0,3 S;
d) une masse moléculaire déterminée par ultracentrifugation de 35 000 ± 5000;
e) un point isoélectrique de pH 3,4 ± 0,4;
f) un coefficient d'extinction $E_{1 cm}^{1\%}$ (280 nm) de 1,9 ± 0,3;
g) une mobilité électrophorétique dans le domaine compris entre les $\alpha_1$ et les $\alpha_2$-globulines;
h) une réaction immunologique spécifique avec un anticorps spécifiquement opposé à la glycoprotéine.

2. Procédé de concentration de la glycoprotéine selon la revendication 1, caractérisé en ce qu'on soumet une solution de protéines qui contient la glycoprotéine, à au moins une des opérations suivantes et on récupère la fraction enrichie en la glycoprotéine concernée:
a) addition de sels neutres jusqu'à précipitation de la glycoprotéine;
b) fractionnement au tamis moléculaire et récupération de la fraction ayant une masse moléculaire de 25 000 à 75 000;
c) adsorption de la glycoprotéine sur un échangeur d'ions faiblement basique et élution de la glycoprotéine;
d) addition de dérivés solubles dans l'eau d'une base d'acridine ou de quinoléine dans une gamme de pH de 5 à 10 jusqu'à une concentration finale d'environ 0,8%;
e) traitement de la solution de glycoprotéine par l'hydroxylapatite;

f) électrophorèse préparative en zones et récupération de la zone entre les $\alpha_1$ et les $\alpha_2$-globulines;
g) traitement de la solution de protéines avec un immunoadsorbant.

3. Procédé selon la revendication 2, caractérisé en ce que comme solution de protéines, on utilise un extrait de placentas humains.

4. Utilisation de la protéine selon la revendication 1 pour l'obtention d'antisérums.

5. Antisérum obtenu par immunisation de vertébrés avec une glycoprotéine selon la revendication 1.

**Claims**

1. Glycoprotein, characterized by
a) a protein proportion of 75 ± 6%;
b) a carbohydrate proportion of 24,6 ± 5,2%; of which 8,9 ± 2% of hexoses, 7,1 ± 1,5% N-acetylated hexosamine, 0,2 ± 0,2% of fucose and 8,4 ± 1,5% of N-acetylated neuraminic acid;
c) a sedimentation coefficient $S_{20w}$ of 2,5 ± 0,3 S;
d) a molecular weight of 35 000 ± 5000 determined in the ultracentrifuge;
e) an iso-electric point of pH 3,4 ± 0,4;
f) an extinction coefficient $E_{1 cm}^{1\%}$ (280 mm) of 1,9 ± 0,3;
g) an electrophoretic mobility in the range between $\alpha_1$- and $\alpha_2$-globulines;
h) a specific immunologic reaction with an antibody directed specifically against the glykoprotein.

2. Process for enriching the glycoprotein defined in claim 1, which comprises subjecting a protein solution containing that glycoprotein to at least one of the following measures and isolating the fraction enriched with that glycoprotein:
a) addition of neutral salts until the glycoprotein is precipitated;
b) molecular sieve fractionation and isolation of the fraction having a molecular weight of between 25 000 and 75 000;
c) adsorption of the glycoprotein on a weakly basic ion exchanger and elution therefrom;
d) addition of water-soluble derivatives of an acridine or quinoline base in a pH-range of from 5 to 10 until a final concentration of about 0,8% is reached;
e) treatment of the glycoprotein solution with hydroxyl-apatite;
f) preparative zone electrophoresis and isolation of the zone between $\alpha_1$- and $\alpha_2$-globulines;
g) treatment of the glycoprotein solution with an immuno adsorbant.

3. A process as claimed in claim 2, wherein said protein solution is an extract of human placentas.

4. The use of the protein defined in claim 1 for the preparation of antisera.

5. An antiserum obtained by immunization of vertebrates with the glycoprotein defined in claim 1.